# EUROPÄISCHE PATENTSCHRIFT

(11) **EP 2 747 835 B1**
(45) Veröffentlichungstag und Bekanntmachung des Hinweises auf die Patenterteilung: **24.02.2021**
(21) Anmeldenummer: 12777847.0
(22) Anmeldetag: 16.08.2012
(51) Int. Cl.: A61N 1/05, A61N 1/362, A61N 1/39

(54) **IMPLANTIERBARE EPIKARDIALE ELEKTRODEN ANORDNUNG**
IMPLANTABLE EPICARDIAL ELECTRODE ARRANGEMENT
ENSEMBLE D'ÉLECTRODES ÉPICARDIALES IMPLANTABLES

(30) Priorität: 26.08.2011 DE 102011111649
(43) Veröffentlichungstag der Anmeldung: 02.07.2014
(73) Patentinhaber: Osypka, Peter, 79639 Grenzach-Wyhlen (DE)
(72) Erfinder: Osypka, Peter, 79639 Grenzach-Wyhlen (DE)
(86) Internationale Anmeldenummer: PCT/DE2012/000825
(87) Internationale Veröffentlichungsnummer: WO 2013/029587

(56) Entgegenhaltungen:
- US-A1- 2007 043 416
- US-A1- 2007 106 336
- US-A1- 2007 106 359
- US-B1- 7 640 065

## Beschreibung

### Die Erfindung:

Die Erfindung betrifft Ausführung und Fixierung einer implantierbaren epikardialen Elektroden Anordnung, die eine Messung, Überwachung, Stimulation und Defibrillation (Kardioversion) des Herzens beim Menschen in Verbindung mit einem außerhalb des Körpers anschließbaren Herzschrittmacher oder Defibrillator ermöglicht und nach erfolgter Anwendung jederzeit leicht durch Zug entfernbar ist, wobei die auf dem Herzen fixierten Elektroden großflächig und multipolar ausgebildet sind und gleichzeitig als Sensing- und Stimulationselektroden verwendet werden können.

### Problem:

Vorhofflimmern tritt mit zunehmendem Lebensalter und nach Herzoperationen relativ häufig auf und ist einer der wichtigsten Gründe für die postoperative Morbidität. Insgesamt scheint Vorhofflimmern im Allgemeinen und besonders nach Herzoperationen in den letzten Jahren zugenommen zu haben, was mit einem immer älter werdenden Patientenkollektiv erklärt wird. Literaturangaben über postoperatives Vorhofflimmern bei Patienten mit bisherigem Sinusrhythmus ergaben ein durchschnittliches Auftreten in 30% - 40% nach Bypass-Operationen am Herzen.

Vorhofflimmern führt zu einer schnellen Überleitung der Erregung auf die Ventrikel, so dass es zu akuter hämodynamischer Instabilität kommen kann. Die bisherige angewandte elektrische Kardioversion ist eine nicht-medikamentöse und sehr effektive Methode zur Wiederherstellung des Sinusrhythmus, die allerdings eine Kurznarkose zur Voraussetzung hat. Diese Kurznarkose kann jedoch gerade bei Patienten nach Bypass-Operation die bestehenden neuronalen Probleme (Vigilanz) durch die soeben überstandene Herzoperation verschärfen, was zu einer verlängerten Aufwachphase führen kann oder sogar eine erneute Intubation mit maschineller Beatmung erforderlich machen. Ein weiteres Problem stellt die Antikoagulation bei postoperativen Patienten mit Vorhofflimmern dar. Wenn die Rhythmusstörung länger als 24 Stunden anhält, ist eine Antikoagulation erforderlich, um die Thrombenbildung mit der Gefahr eines Schlaganfalls zu reduzieren. All diese Faktoren führen zu einem komplizierten postoperativen Verlauf bei Patienten nach Bypass-Operation, der sich in einem um ca. 5 Tage verlängerten Krankenhaus Aufenthalt mit vermehrten Kosten niederschlägt. Vorhofflimmern kann während der Liegedauer des Patienten auf der Intensivstation wiederholt auftreten. Zur Prophylaxe wird in der Literatur das sogenannte "Multisite Pacing" beschrieben. Hierbei wird besonders der linke Vorhof an mehreren Arealen gleichzeitig stimuliert. Für das "Multisite Pacing" werden z.B. unipolare Herzdrähte verwendet, die gegenüber einer äußeren Anode betrieben werden.

Durch die anatomische Lage des linken Vorhofs ist die Fixierung von mehreren Elektroden dort besonders erschwert, was sich durch häufigen Stimulationsausfall bei der Anwendung bemerkbar macht. Eine echte Ursachenforschung für das erneute Auftreten von Vorhofflimmern gibt es aus Mangel an geeigneten Elektroden bisher nicht.

Wie wird das Problem bisher gelöst:
Die Beseitigung von Vorhofflimmern erfolgt bisher durch einen äußeren elektrischen Energieimpuls mittels eines Defibrillators, appliziert durch Auflage oder Aufkleben von großflächigen Elektroden auf den Brustkorb des Patienten. Dazu ist es notwendig, dass beim Patienten vorher eine Ultraschalluntersuchung des linken Herzohres durchgeführt werden muss und während der Kardioversion eine Narkose notwendig ist. In der Literatur werden auch Methoden nach Herzoperationen beschrieben, die mit jeweils einer blanken Litze auf den Vorhöfen und dann zwischen den Vorhöfen die Kardioversion stattfindet. Die dafür notwendigen Schockenergien liegen zwischen 5 - 9 Joule und verursachen weiterhin erhebliche Schmerzen.

Es besteht daher die Aufgabe, Elektroden nach der eingangs beschriebenen Art zu schaffen, die eine gute Verankerung und eine stabile temporäre Stimulation auch für eine mehrfache gleichzeitige Stimulation an verschiedenen Arealen des Herzens (Multisite Pacing) ermöglichen oder eine schmerzfreie Beseitigung des Vorhofflimmerns des Herzens durch Kardioversion und eine Prophylaxe zur Verhinderung von wiederholtem Auftreten von Vorhofflimmern zu ermöglichen.

Diese Aufgabe wird mit den Mitteln und Merkmalen des Schutzanspruches 1 gelöst. Dadurch dass die Kardioversion mit Elektroden, die temporär an den beiden Vorhöfen epikardial und lokal fixiert sind, vorgenommen wird, reduziert sich die notwendige elektrische Energieabgabe zur Kardioversion erheblich.

Durch Einsatz von Elektroden entsprechend Schutzanspruch 1 ergibt sich die Möglichkeit einer unmittelbaren Beseitigung und Unterdrückung von erneutem Auftreten des Vorhofflimmerns ohne Narkose für den Patienten. Später lassen sich diese Elektroden - wie bei den üblich heute benutzten Herzdrähten - leicht entfernen.

Temporäre Myokardiale Elektroden (auch als Herzdrähte bekannt), ermöglichen nach einer Herzoperationen eine externe Stimulation des Herzens. Derartige Elektroden sind seit vielen Jahren bekannt und werden routinemäßig nach jeder offenen Herzoperation zur Stimulation der Vorhöfe und Ventrikel eingesetzt. Die Fixierung am Herzen muss so erfolgen, dass einerseits die Elektroden während der Zeit auf der Intensivstation stabil fixiert sind, andererseits müssen sie sich leicht danach durch eine kleine Öffnung in der Bauchdecke des Patienten nach außen herausziehen lassen.

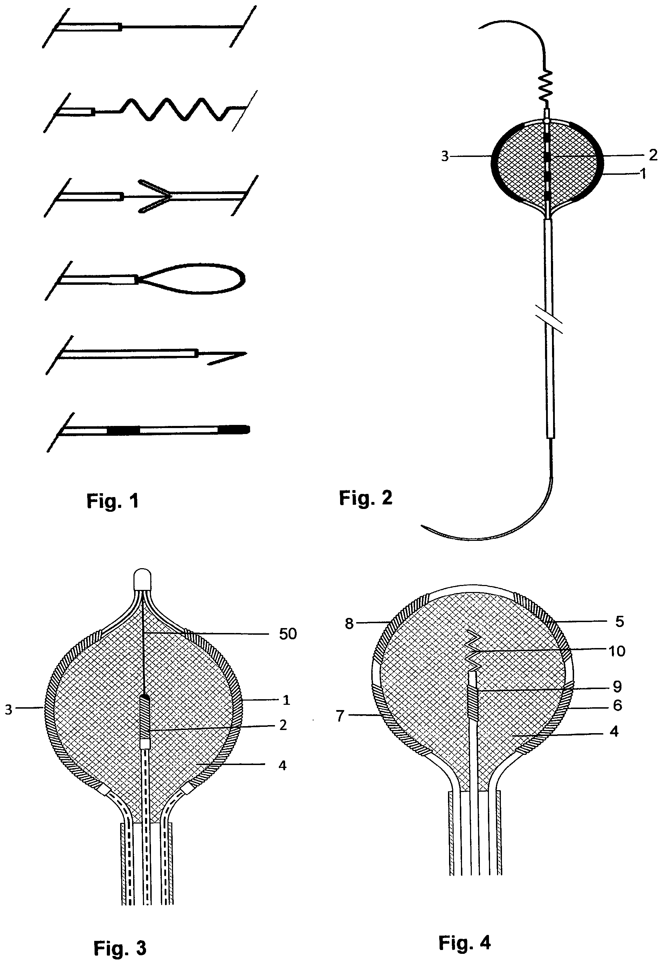

Für die temporäre Fixierung der Elektroden im Herzmuskel sind in Fig.1 die verschiedenen heute gebräuchlichen Methoden dargestellt. Fixierungsarten bei denen nach der Entfernung der Elektroden Nahtmaterial im Herzmuskel verbleibt werden nicht immer akzeptiert, insbesondere bei kleinen Herzen von Kindern. Auch die Zick-Zack Fixierung und die Kunststoffwendel sind für derartige Anwendungen oft zu groß. Sehr gern wird die Fixierung mit dem Kunststoffanker eingesetzt. Der Nachteil hierbei ist die unbestimmte Fläche der Elektrode. Der Anker wird aus der Isolierung der Litze in drei Streifen herausgeschnitten. Der Rest der übrig bleibenden Litze bildet den differenten Pol. Da diese Fläche sehr klein ist, bewirkt jede kleine physische Bewegung eine Reizschwellenänderung Aus US2007106359 A1 ist eine korsett-förmige epikardiale Elektrodenanordnung zur Defibrillation und Stimulation mittels einer externen Spannungsquelle bekannt.

Es besteht daher die Aufgabe, eine Fixierung von Herzdrähten nach der eingangs beschriebenen Art zu schaffen, die eine gute Verankerung und eine stabile temporäre Stimulation des Herzens ermöglicht.

### Abbildungen:

Weitere Einzelheiten, Merkmale und Vorteile der Erfindung lassen sich dem folgenden Beschreibungsteil entnehmen, in dem anhand der Zeichnungen und Ausführungsbeispiele die Erfindung näher erläutert wird.

Sie zeigen in schematischer Darstellung in
Fig.1 zeigt verschiedene distale Enden der heute gebräuchlichen temporären Elektroden für die Stimulation des Herzens nach einer Herzoperation in Form von Metalllitze mit unterschiedlichen Fixierungen.
Fig.2 zeigt eine beispielhafte Elektrodenanordnung für die Defibrillation (Kardioversion) und Stimulation des Herzens in Form eines Tennisschlägers.
Fig.3 zeigt eine weitere beispielhafte Elektrodenanordnung für die Defibrillation (Kardioversion) und Stimulation des Herzens mit in Form eines Tennisschlägers angeordneten Elektroden in Form von Metallwendeln, dessen gesamte, dem Herzen abgewandte Fläche, isoliert ist.
Fig.4 zeigt eine weitere beispielhafte Elektrodenanordnung für die Defibrillation (Kardioversion) und gleichzeitige Stimulation an unterschiedlichen Stellen des Herzens in Form von kreisförmig angeordneten Metallwendeln, dessen Außen Fläche ebenfalls isoliert ist.

Fig.5 zeigt eine weitere beispielhafte Elektrodenanordnung für die Defibrillation (Kardioversion) und multipolare Stimulation an unterschiedlichen Stellen des Herzens mit in Form eines Hexagons angeordneten Wendeln, die auf der dem Herzen abgewandten Seite zusätzliche Noppen aufweist, um ein Verrutschen der Elektrode zu verhindern und die auf der dem Herzen zugewandten Seite ein Array von Elektroden enthält, die die Areale des Herzen elektrisch abtasten (Mapping) und stimulieren können.
Fig. 6 zeigt eine weitere beispielhafte Elektrodenanordnung für die Defibrillation (Kardioversion) und multipolare Stimulation an unterschiedlichen Stellen des Herzens in Form von kreisförmig angeordneten Außen Wendeln (14,15), die in einem Silikonschlauch (39) integriert sind, der auf der dem Herzen zugewandten Seite Öffnungen (40) aufweist.
Fig.7 zeigt eine weitere beispielhafte Elektrodenanordnung für die Defibrillation (Kardioversion) und multipolaren Stimulation an unterschiedlichen Stellen des Herzens in Form von kreisförmig angeordneten Außen Wendeln und vier inneren positionierbaren Polen, die vertieft angeordnet sind, um eine bessere Fixierung auf der Oberfläche des Herzens zu erreichen.
Fig. 8 zeigt eine weitere beispielhafte Elektrodenanordnung für die multipolare Stimulation an unterschiedlichen Stellen des Herzens die aus Silikonkautschuk gefertigt ist und eine Reihe von Polen aufweist (31).
Fig. 9 zeigt eine weitere beispielhafte Elektrodenanordnung (32), die ebenfalls aus Silikon besteht und mehrere Pole (41) aufweist.
   Um die Reizschwelle niedrig zu halten, kann bei Bedarf der sich hinter dem Elektrodenareal befindlichen Ballon (33) gefüllt werden. Dabei werden die Pole wunschgemäß gegen die Herzwand gedrückt.
Fig.10 zeigt eine weitere beispielhafte Elektrodenanordnung (35), die ebenfalls aus Silikon besteht und mehrere Pole (41) aufweist. Zur besseren Platzierung im Herz ist hier eine taschenförmige Ausbuchtung (34) vorgesehen, die mit Hilfe eines geeigneten Schiebers (Löffel) aus Kunststoff oder Metall die Elektrode in die gewünschte Position dirigieren kann.
Fig.11 zeigt eine weitere beispielhafte Elektrodenanordnung für die Defibrillation (Kardioversion) und multipolaren Stimulation an unterschiedlichen Stellen des Herzens in Form von kreuzförmig angeordneten Elektroden Polen.
Fig. 12 zeigt eine weitere beispielhafte Elektrodenanordnung für die Defibrillation (Kardioversion) und multipolare Stimulation an unterschiedlichen Stellen des Herzens in Form von sternförmig angeordneten Elektroden Polen.
Fig.13 zeigt eine weitere beispielhafte Elektrodenanordnung für die Defibrillation (Kardioversion) und multipolare Stimulation an unterschiedlichen Stellen des Herzens in Form von elliptisch ähnlichen angeordneten Elektroden Polen.
Fig.14 zeigt eine weitere beispielhafte Elektrodenanordnung für die Defibrillation (Kardioversion) und multipolare Stimulation an unterschiedlichen Stellen des Herzens in Form von fächerförmig angeordneten Elektroden Polen.
Fig. 15 zeigt eine beispielhafte Darstellung einer an der Außenseite des menschlichen Körpers angebrachten elektrischen und mechanischen Verbindung zwischen der Inneren Elektroden Anordnung und den anzuschließenden Medizinischen Geräten.
Fig.16 zeigt eine beispielhafte Darstellung einer an der Außenseite des menschlichen Körpers angebrachte Telemetrie Einrichtung, die die aus dem Herzen gemessenen Daten weiter senden kann, die aber auch von außen gesendete Daten, wie elektrische Impulse etc. an die Elektroden Anordnung weiter leiten kann. Diese Telemetrie Einrichtung kann auch subkutan implantiert werden.

### Beschreibung der Abbildung:

Fig.1 zeigt heute gebräuchliche temporäre Elektroden für die Stimulation des Herzens nach einer Herzoperation in Form von Metalllitze mit unterschiedlichen Fixierungen.

Fig.2 zeigt eine beispielhafte Elektrodenanordnung für die Kardioversion und Stimulation des Herzens in Form eines Tennisschlägers mit großflächig angeordneten Metallwendeln (1,3). Neben ihrer Flexibilität und Anordnung vergrößern sie vorteilhaft die Elektroden Oberfläche oder können sich der Anatomie des Herzens sehr gut anpassen. Zwischen diesen beiden voneinander isolierten Polen der Wendel (1,3) kann eine Defibrillation erfolgen. Die Stimulation des Herzens kann zwischen den einzelnen Polen (2) und der Wendel (1), zwischen den Polen (2) und Wendel (3), oder zwischen den einzelnen Polen (2) untereinander, erfolgen.

Wichtig ist, dass bei der erfindungsgemäßen Anordnung aufgrund der großflächig angeordneten Elektroden (Metallwendeln) die Defibrillation separat an jedem Vorhof erfolgen kann. Ferner ist die Form der Elektrodenanordnung wesentlich um die Defibrillations-und die Stimulationselektroden in einer Anordnung möglich zu machen, denn nur dadurch wird das Problem der schwierigen Fixierung der Elektroden gelöst.

Die für die Defibrillation benötigten Schockenergien liegen unter 5 Joule, vorzugsweise unter 2 Joule, besonders bevorzugt unter 1 Joule, sodass die Defibrillation weitgehend schmerzfrei erfolgen kann.

Ebenso erlaubt die erfindungsgemäße Anordnung eine Stimulation ohne dass eine weitere Stimulationselektrode fixiert werden muss.

An unterschiedlichen Ausführungsbeispielen soll die Vielfalt der erfindungsgemäßen Elektrode beschrieben werden.

Der Aufbau der Elektrodenpole kann neben einer Wendel aus einfach oder mehrfach parallel gewickelten Drähten, Bändern, blank oder teilweise isoliert mit unterschiedlichen Durchmessern und Materialien bestehen. Auch Silikonfolien mit einer Anzahl von leitfähigen Polen aus Metall oder Kunststoff, die an das Zielgebiet angepasst sind, kommen zur Anwendung. Weiterhin kann die Elektrode aus Drahtgeflecht, Metalllitze oder parallelen Leitern aufgebaut sein.

Unterschiedliche Ausgestaltung der erfindungsgemässen Elektrodenanordnung: Materialauswahl: Edelstahl, Platin, Gold, Elgiloy, Nitinol, Isotan, elektrisch leitfähige Fäden, Karbonfäden, elektrisch leitfähiger Kunststoff, auch mit Nanopartikeln gemischt.
Isolation: Polyurethan, Polyethylen, Silikon, PTFE, Pebax, Polyamid, Peek, alle biokompatiblen Kunststoffe incl. Lacke, z.B. Polyimid,
Aufbau: Wendel, Geflecht, Litze, Draht, Kunststoff, Metallbänder, Metallrohr, Karbonfasern, jeweils den Herzkonturen angepasst, sodass unterschiedliches Drahtmaterial, Drahtstärken und Anzahl der Drähte und Kunststofffäden in Betracht gezogen werden kann.
Form der Anordnung: kreisförmig, tennisschlägerartig, elliptisch, Hexagonal, rechteckig, eichenblattförmig, sternförmig, kleeblattförmig,
Form der Pole: Hülse, Zylinder, Kugel, Olivenförmig, Halbkugel, Wendel, Spirale, Pilzform,
Fixierung am Herzen: Fäden, Ösen, Haken, Zick-Zack, Wendel, Spirale, Chirurgisches Nahtmaterial, weiche Kunststoff Noppen z.B. aus Silikon,
Schaft - Zuleitung: Litze, Draht, Wendel, Kanüle, isoliert oder in Schutzschläuchen,

Fig.3 zeigt eine weitere beispielhafte Elektrodenanordnung für die Kardioversion und Stimulation des Herzens in Form eines Tennisschlägers mit angeordneten Metallwendeln (1). Damit die bei der elektrischen Stimulation/Defibrillation auftretende Feldstärkeverteilung ausschließlich in die Richtung des Herzens wirksam wird, wird der dem Herzen abgewandte Teil der Elektrodenanordnung durch eine dünne Kunststoffmembran (4) isoliert. Als Isolationsmaterial eignet sich besonders eine Folie aus Silikon. Der differente Elektroden Pol (2) ist zur optimalen Positionierung auf dem Faden (50) verschiebbar und anschließend fixierbar (hier nicht dargestellt).

Fig.4 zeigt eine weitere beispielhafte Elektrodenanordnung für die Kardioversion und Stimulation des Herzens in Form eines Tennisschlägers mit angeordneten Metallwendeln (5-8). Durch die tennisschlägerförmige Anordnung der isolierten Polen ergibt sich eine Vielzahl von möglichen Arealen auf dem Herzen, die man programmiert stimulieren kann, um so das Entstehen von Vorhofflimmern zu verhindern. Auch hier kann die dem Herzen abgewandte Seite der Elektrodenanordnung durch eine Kunststoffmembran (4) isoliert werden. Die Elektrodenanordnung auf dem Herzen muss einerseits bei Gebrauch eine stabile Position haben, andererseits muss die Anordnung nach ein paar Tagen leicht allein durch Zug durch eine kleine Öffnung im Brustraum entfernt werden können. Hierzu dient die Wendelform des Pols (10), dessen Wendel so gestaltet ist, dass die einzelnen Windungen im Durchmesser größer sind als die Pole 5-8. Dadurch dass die Silikonmembran einseitig auf die vergrößerte Wendel (10) drückt, wird diese gegen das Gewebe des Herzens gedrückt und damit eine Positions- Änderung weitgehend verhindert.

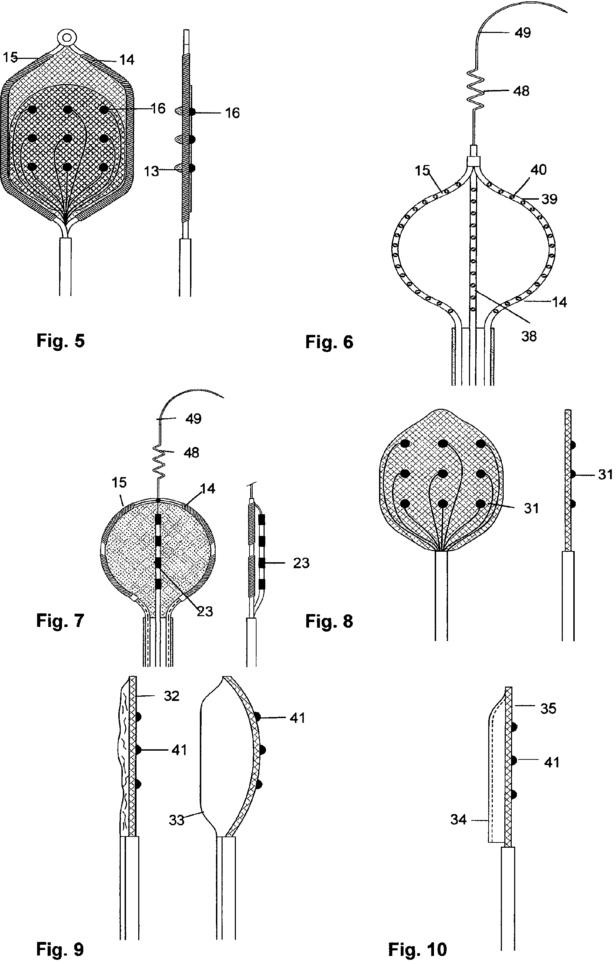

Fig.5 zeigt eine weitere beispielhafte Elektrodenanordnung für die Kardioversion und zur multipolaren Stimulation an unterschiedlichen Stellen des Herzens mit in Form eines Hexagons angeordneten Wendeln (14,15), die auf der dem Herzen abgewandten Seite zusätzliche Noppen (13) (z.B. aus Silikon) aufweisen, um ein Verrutschen der Elektrode zu verhindern und die auf der dem Herzen zugewandten Seite ein Array von Elektroden (16) enthält, die die Areale des Herzen elektrisch abtasten (Mapping) oder stimulieren können.

Fig.6 zeigt eine weitere beispielhafte Elektroden Anordnung für die Kardioversion und zur multipolaren Stimulation an unterschiedlichen Stellen des Herzens in Form von kreisförmig angeordneten Außen Wendeln (14,15), die in einem Silikonschlauch (39) integriert sind, wobei der Silikonschlauch auf der dem Herzen zugewandten Seite Öffnungen (40) aufweist. Die Löcher sind in Form, Abstand und Größe so ausgebildet, dass sie als Stimulations-oder Defibrillations- Elektrode voll wirksam sind. Auch der innere Teil der Elektroden Anordnung wird durch einen gelochten Silikonschlauch (38) so umhüllt, dass die einzelnen Pole in Anzahl und Größe auf der dem Herzen zugewandten Seite voll wirksam sind. Die gebogene Herznadel (49) und der zick-zack-förmige Faden (48) dienen zur Fixierung der epikardialen Anordnung auf dem Herzen.

Die Zuleitungen zu den Elektroden können sowohl parallel, koaxial oder übereinander angeordnet sein.

Fig.7 zeigt eine weitere beispielhafte Elektrodenanordnung für die Kardioversion und zur multipolaren Stimulation an unterschiedlichen Stellen des Herzens in Form von kreisförmig angeordneten Außen Wendeln (14,15) und vier inneren positionierbaren Polen (23), die vertieft angeordnet sind, um eine bessere Fixierung auf der Oberfläche des Herzens zu erreichen. Die gebogene Herznadel (49) und der zick-zack-förmige Faden (48) dienen zur Fixierung der epikardialen Anordnung auf dem Herzen.

Fig. 8 zeigt eine weitere beispielhafte Elektrodenanordnung für die multipolare Stimulation an unterschiedlichen Stellen des Herzens die aus Silikonkautschuk gefertigt ist und eine Reihe von Polen ((31) aufweist. Aufgrund ihrer Flexibilität durch das biokompatible Silikon, lassen sich derartige Elektroden zur Überwachung und Stimulation leicht während einer Operation auch unter das Herz schieben. Die Stimulationselektrode kann separat verwendet werden, kann aber auch wie in Fig. 5 dargestellt Teil der erfindungsgemäßen Elektrodenanordnung sein.

Fig. 9 zeigt eine weitere beispielhafte Elektrodenanordnung (32), die ebenfalls aus Silikon besteht und mehrere Pole (41) aufweist. Um die Reizschwelle niedrig zu halten, kann bei Bedarf der sich hinter dem Elektrodenareal befindlichen Ballon (33) gefüllt werden. Dabei werden die Pole wunschgemäß gegen die Herzwand gedrückt.

Fig.10 zeigt eine weitere beispielhafte Elektrodenanordnung (35) die ebenfalls aus Silikon besteht und mehrere Pole (41) aufweist. Zur besseren Platzierung im Herz ist hier eine taschenförmige Ausbuchtung (34) vorgesehen, die mit Hilfe eines geeigneten Schiebers (Löffel) aus Kunststoff oder Metall die Elektrode in die gewünschte Position dirigieren kann.

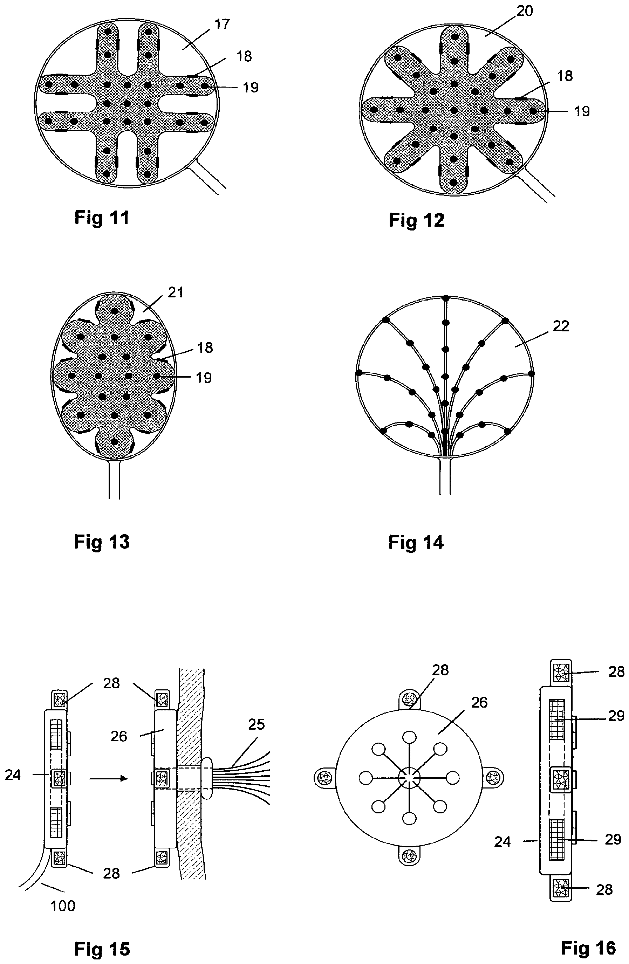

Fig.11 zeigt eine weitere beispielhafte Elektrodenanordnung für die Kardioversion und zur multipolaren Stimulation an unterschiedlichen Stellen des Herzens in Form von kreuzförmig (17) angeordneten Elektroden Polen (18,19).

Fig.12 zeigt eine weitere beispielhafte Elektrodenanordnung für die Kardioversion und zur multipolaren Stimulation an unterschiedlichen Stellen des Herzens in Form von sternförmig angeordneten Elektroden Polen (20).

Fig.13 zeigt eine weitere beispielhafte Elektrodenanordnung für die Kardioversion und zur multipolaren Stimulation an unterschiedlichen Stellen des Herzens in Form von elliptisch ähnlichen angeordneten Elektroden Polen (21).

In Fig. 11-13 dargestellten Elektrodenanordnungen können separat als Stimulationselektroden verwendet werden. Sie können aber auch Teil der erfindungsgemäßen Anordnung sein. Es ist möglich eine solche Stimulationselektrode zwischen kreisförmig angebrachte Defibrillationselektroden zu installieren. Auch kann der Pol 18 als Wendel eichenblattförmig ausgebildet sein.

Fig.14 zeigt eine weitere beispielhafte Elektrodenanordnung zur multipolaren Stimulation an unterschiedlichen Stellen des Herzens in Form von fächerförmig angeordneten Elektroden Polen (22). Diese Stimulationselektrode kann auch Teil der erfindungsgemäßen Elektrodenanordnung sein.

Die bisherigen Darstellungen zeigen, dass die Erfindung nicht allein auf diese Formen beschränkt ist, sondern die Gestaltung leicht veränderbar und an die anatomischen Gegebenheiten anpassbar ist.

Fig. 15 zeigt eine beispielhafte Darstellung einer an der Außenseite des menschlichen Körpers angebrachten elektrischen und mechanischen Verbindung zwischen der Inneren Elektroden Anordnung (25) und den anzuschließenden Medizinischen Geräten (24). Die vom Herzen kommenden Elektroden (25) werden nach außen zu einer flexiblen Kunststoffscheibe (26) geführt, die auf der Haut des Patienten fixiert werden kann. Durch die in der Scheibe dargestellten 8 Löcher kann die Scheibe elastischer gestaltet werden. An vier Stellen der Scheibe (26) und der abnehmbaren Anschlusskappe(24) sind Magnete (28) integriert, die dafür sorgen, dass die Anschlusskappe (24) elektrisch und mechanisch fest mit der Kunststoffscheibe (26) verbunden wird. Die Verbindung zu den externen Geräten (Stimulator, Defibrillator) erfolgt über eine Kabelverbindung 100.

Fig. 16 zeigt eine beispielhafte Darstellung einer an der Außenseite des menschlichen Körpers angebrachten Telemetrie Einrichtung (29), beispielsweise eine Spule, die in der Anschlusskappe (24) integriert ist, die die aus dem Herzen gemessenen Daten weiter senden oder speichern kann, die aber auch von außen gesendete Daten, wie elektrische Impulse oder andere medizinische Daten an die Elektroden weiter leiten kann. Diese Telemetrie Einrichtung kann auch subkutan implantiert werden. Telemetrie Einrichtungen zur Übertragung medizinische Daten sind heute Stand der Technik. Es wurde daher auf eine weitere Erklärung und Funktionsweise verzichtet.

## Patentansprüche

1. Implantierbare epikardiale Elektrodenanordnung, die eine Messung, Überwachung, Stimulation und Defibrillation (Kardioversion) des Herzens beim Menschen in Verbindung mit einem außerhalb des Körpers anschließbaren Herzschrittmacher oder Defibrillator ermöglicht, wobei die auf dem Herzen fixierten Elektroden großflächig und multipolar ausgebildet sind und gleichzeitig als Sensing- und Stimulationselektroden verwendet werden können;
wobei die Elektrodenanordnung aus mindestens zwei großflächig angeordneten Elektroden (1,3),
einer weiteren Elektrode (2) die zwischen den großflächig angeordneten Elektroden (1,3) angeordnet ist, wobei die Elektrode (2) multipolar ist und die Stimulation des Herzens zwischen den einzelnen Polen der multipolaren Elektrode erfolgen kann. und einer Fixierungsvorrichtung besteht,
wobei zwischen den voneinander isolierten Polen der Elektroden (1,3) die Defibrillation erfolgen kann und wobei zwischen einer der Elektroden (1 oder 3) und einem Pol der Elektrode (2) die Stimulation des Herzens erfolgen kann, **dadurch gekennzeichnet, dass** die Elektrodenanordnung die Form eines Tennischlägers besitzt.

2. Implantierbare epikardiale Elektrodenanordnung gemäß Anspruch 1, wobei die Elektroden (1,3) und die Elektrode (2) aus einem Metallgeflecht, Metalllitze oder aus einer Metallwendel bestehen.

3. Implantierbare epikardiale Elektrodenanordnung gemäß Anspruch 1 bis 2, wobei der vom Herzen abgewandte Teil der Elektrodenanordnung durch eine dehnbare isolierende Folie, vorzugsweise Silikonfolie, abgedeckt ist.

4. Implantierbare epikardiale Elektrodenanordnung gemäß einem der Ansprüche 1 bis 3, wobei die Elektroden (1, 3) und (2) in einem isolierenden hochflexiblen Schlauch integriert sind, der für jeden Pol mit mindestens einer Öffnung versehen ist, die in Gebrauchsstellung in Richtung Herz zeigt.

5. Implantierbare epikardiale Elektrodenanordnung gemäß Anspruch 4, wobei die Pole der Elektrode in ihrem Abstand veränderbar sind und durch eine Öffnung des Schlauches auch Flüssigkeiten oder Medikamente transportiert werden können.

6. Implantierbare epikardiale Elektrodenanordnung gemäß einem der Ansprüche 1 bis 5, wobei die Fixierung der Elektrode auf der äußeren Herzwand durch unterschiedliche Ankerformen mittels Fäden, Wendel oder Loops vorgenommen werden kann.

7. Implantierbare Elektroden nach Anspruch 1 bis 6,**dadurch gekennzeichnet**, das sämtliche Informationen, Signale und die zugeführte Energie für die Stimulation oder Kardioversion des Herzens, gespeichert und drahtlos in beiden Richtungen übertragen werden können.

## Claims

1. An implantable epicardial electrode assembly used in conjunction with an external pacemaker or defibrillator positioned outside of the body and permitting measurement, monitoring, stimulation and defibrillation (cardioversion) of the human heart; the assembly comprises electrodes configured to be fixed on the heart, whereby the electrode poles have a large surface area and are multipolar respectively and may be used at the same time as sensing electrodes and as stimulation electrodes;
the assembly comprises at least two electrode poles (1,3) of large scale and a further electrode (2) arranged between the electrode poles (1,3) whereby electrode (2) is multipolar; and
the electrode assembly further comprises a fixing element; and
whereby defibrillation may occur between the isolated poles of electrode (1,3) and stimulation of the heart may occur between the individual poles of the multipolar electrode (2) or between one of the electrode poles (1 or 3) and one of the poles of electrode (2)
**characterized in that** the electrode assembly is racket-shaped.

2. The implantable epicardial electrode assembly according to claim 1, wherein the electrode poles (1,3) and the poles of electrode (2) comprise a metal mesh, metal strand or a metal filament.

3. The implantable epicardial electrode assembly according to claim 1 or 2, wherein the part of the electrode assembly facing away from the heart is covered by a stretchable insulating film which comprises silicone.

4. The implantable epicardial electrode assembly according to any one of claims 1-3, wherein the electrode poles (1,3) and electrode pole (2) are integrated into an insulating highly flexible tube said tube having at least one opening for each pole, wherein the opening faces in use position towards the heart.

5. The implantable epicardial electrode assembly according to claim 4, wherein the distance between the electrode poles may be varied and wherein liquids or liquid medicine can flow through an opening.

6. The implantable epicardial electrode assembly according to any one of claims 1-5,
wherein the fixing element has various anchor shapes including threads, helix, or loops.

7. The implantable epicardial electrode assembly according to any one of claims 1-6,
wherein all information signals and supplied power for pacing or cardioversion of the heart is stored, and can be wirelessly transmitted in both directions.

## Revendications

1. Ensemble d'électrodes épicardiales implantables permettant la mesure, la surveillance, la stimulation et la defibrillation (cardioversion) du cœur humain en contact avec un stimulateur cardiaque ou un défibrillateur qui peut être connecté à l'extérieur du corps; l'ensemble comprend les électrodes épicardiales qui sont fixées sur la surface du coeur; les pôles d'électrodes ont une grande surface et sont multipolaires et peuvent être utilisées simultanément comme des électrodes de détection et de stimulation; l'ensemble d'électrodes comprend au moins deux électrodes de grande surface (1, 3) et une autre électrode (2) qui est disposée entre les électrodes (1, 3); laquelle électrode (2) est multipolaire; l'ensemble comprend aussi un dispositif de fixation; et
les pôles d' électrodes (1, 3) qui sont isolés électriquement l' un de l'autre sont prêts à défibriller; et le cœur peut être stimulé entre les pôles individuels de l'électrode (2) ou entre l'une des électrodes (1 ou 3) et un pôle de l'électrode (2);
**caractérisé en ce que** l'ensemble d'électrodes a la forme d'une raquette de tennis.

2. Ensemble d'électrodes épicardiales implantables selon la revendication 1, dans lequel les électrodes (1, 3) et l'électrode (2) sont constituées d'un tresse métallique, d'une toron métallique ou d'une hélice métallique.

3. Ensemble d'électrodes épicardiales implantables selon la revendication 1 ou 2, dans lequel la partie qui se trouve opposée au cœur, est couverte d'un film isolant et élastomère, de préférence d'un film de silicone.

4. Ensemble d'électrodes épicardiales implantables selon la revendication 1 à 3 dans lequel les électrodes (1, 3) et l'électrode (2) se trouvent dans un tube isolant élastique, le tube a au moins une ouverture pour chaque pôle, qui est en position d'utilisation orienté vers le cœur.

5. Ensemble d'électrodes épicardiales implantables selon la revendication 4, dans lequel la distance entre les pôles peut être modifiée et dans lequel des liquides ou des médicaments peuvent être donnés par une ouverture dans le tube.

6. Ensemble d'électrodes épicardiales implantables selon la revendication 1 à 5, dans lequel l'électrode est fixé epicardiales par un fils, une hélice métallique ou une boucle.

7. Ensemble d'électrodes épicardiales implantables selon la revendication 1 à 6 **caractérisées en ce que** toutes les informations, tous les signaux et l'énergie pour la stimulation ou la cardioversion du coeur peuvent être accumulés et transmis sans fil.
